# EUROPÄISCHE PATENTANMELDUNG

(11) **EP 3 771 547 A1**
(43) Veröffentlichungstag der Anmeldung: **03.02.2021**
(21) Anmeldenummer: 19189755.2
(22) Anmeldetag: 02.08.2019
(51) Int. Cl.: B29C 64/112, B33Y 10/00, C12N 5/00, A61L 27/38, A61L 27/54

(54) **VERFAHREN ZUM 3D DRUCK VON VASKULARISIERTEN GEWEBEN UND ORGANEN**

(71) Anmelder: Technische Universität Berlin, 10623 Berlin (DE)
(72) Erfinder: Schäfer, Dr. Konstanze, 10405 Berlin (DE); Salomon, Andreas, 13505 Berlin (DE)
(74) Vertreter: Hertin und Partner Rechts- und Patentanwälte PartG mbB

(57) **Zusammenfassung**

Ein 3D-Druckverfahren zur Herstellung von vaskularisierten Geweben und Organen bedarf eines Droplet-Druckers zur Erzeugung photorealistischer hochauflösender Drucke und einer Vorrichtung zum Applizieren ungerichteter oder gerichteter elektromagnetischer Wellen. Das Verfahren nutzt eine Kapillartinte mit Zellen und Vernetzungsmolekülen, die nur im Randbereich der Tintentropfen vernetzt oder eine schichtbildende Reaktion durchläuft. Nicht gebundene Kapillartintenbestandteile werden entfernt. Die entstehenden Hohlräume bilden ein Kapillarnetz von Durchmessern bis zu ca.10 µm.

## Beschreibung

Die Erfindung betrifft ein 3D-Druckverfahren zur Herstellung von vaskulären Strukturen aufweisenden Geweben und Organen.

### Einleitung:

Herkömmliche 3D-Drucksysteme besitzen aufgrund ihrer spezifischen Merkmale und der damit einhergehenden Vor- und Nachteile nicht die erforderlichen Eigenschaften, um lebensfähige größere Gewebe und Organe erzeugen zu können.

Um ein größeres lebensfähiges Gewebe oder Organ mittels 3D-Druck herzustellen, muss die Ernährung der gedruckten Zellen gewährleistet sein. Da die Ernährung der Zellen durch Diffusion nur über die Wegstrecke von ca. 500 µm erfolgen kann, ist ein ebenfalls gedrucktes Blutgefäßsystem erforderlich. Zwischen den Arterien und Venen befinden sich das Kapillarsystem, dessen einzelne Kapillargefäße einen sehr geringen Durchmesser von nur ca. 10 µm besitzen und zudem aus einzelligen Zell-Lagen aufgebaut sind.

Bei den bisher bekannten 3D-Drucktechniken handelt es sich entweder um Verfahren, bei denen zwar gleichzeitig verschiedene Zelltypen in einem Schritt gedruckt werden können, die dafür aber die nötige hohe Druckauflösung nicht erreichen (z.B. Fused Deposition Modeling oder Droplet-Technologien), oder um Verfahren, die die erforderliche hohe Druckauflösung erreichen, dafür aber der selektive Druck verschiedener Zelltypen innerhalb eines Arbeitsschritts unmöglich ist (z.B. Stereolithographie-Drucktechniken).

Zusätzlich gibt es spezifische 3D-Drucksysteme wie z.B. den Laser Induced Forward Transfer, bei dem durch einen Laserstrahl einzelne Zellen aus einer Matrix herausgelöst und auf eine Kollektormatrix geleitet werden. Hier kann sowohl eine hohe Druckauflösung erreicht als auch in einem Schritt verschiedene Zelltypen gedruckt werden. Allerdings ist die dabei erreichbare Druckgeschwindigkeit so niedrig, dass diese Technik nur für extrem kleine Strukturen nützlich ist.

Ein anderes spezifisches Drucksystem ist die die Weiterentwicklung des 3D - Stereolithographie - Drucks, bei dem auch verschiedene Zelltypen gedruckt werden können. Hierzu muss aber das zu druckenden Gewebe zwischen Reaktionswannen mit unterschiedlichen Zelltypen gewechselt werden. Dieser Schritt lässt ebenfalls nur sehr wenige Zell-Lagen zu und ist ein relativ langsamer Prozess, mit dem nur kleinere Strukturen wie Miniorgane gedruckt werden können.

Weitere spezifische 3D - Druckverfahren lassen den Raum für spätere Blutgefäße als Hohlraum im gedruckten Gewebe offen und ernähren die umliegenden Zellen zunächst mit Nährmedium. Um echte Kapillaren zu erzeugen, müssen diese Gewebe in einen Organismus überführt werden, der die Hohlräume mit Endothelzellen und diese umgebenden Muskelzellen versorgt. Dieser Schritt kann auch nachträglich in einem Inkubator erfolgen. Wenn auf diese Weise auch letztendlich Kapillaren im Gewebe erzeugt werden können, so dauert der Prozess doch mindestens Tage und trotzdem wird die natürliche Komplexität des betreffenden Gewebes nicht erreicht. Abgesehen davon können mit diesen Techniken nur verhältnismäßig große Gefäßdurchmesser erreicht werden.

Eine weitere Möglichkeit, relativ einfach aufgebaute Gewebe aus langsam wachsenden Zellen wie Knorpel oder Knochen zu erzeugen, ist der 3D-Druck von Scaffolds, die nachträglich mit Zellen besiedelt werden. In diesen langsam wachsenden Gewebetypen bleibt aufgrund des niedrigen Stoffumsatzes genügend Zeit für eine nachträgliche Ausbildung von Kapillaren innerhalb eines Organismus. Dieses Vorgehen ist aber auf Gewebetypen wie Knochen und Knorpel begrenzt. Für schneller wachsende unkomplizierte Zelltypen wie beispielsweise Leberzellen konnte gezeigt werden, dass sehr kleine Gewebebereiche erfolgreich in Leberorgane verpflanzt werden können. Diese Gewebebereiche können aber nur aus sehr kleinen Strukturen bestehen, da die Zellen sonst nicht mehr ernährt werden können.

Alle bisherigen Versuche, funktionstüchtige Gewebe oder Organe mit einem 3D-Drucker zu erzeugen, führten entweder zu sehr kleinen oder sehr langsam wachsenden Gewebeeinheiten oder zu größeren Strukturen, die nur für sehr kurze Zeit lebensfähig waren.

Es bedarf also eines 3D-Druckverfahrens, das
- einerseits innerhalb eines Arbeitsschrittes verschiedene Zelltypen selektiv drucken kann und
- dass andererseits die hohe Druckauflösung von Stereolithographie-Verfahren erreichen kann.
- Zusätzlich soll das Verfahren eine Druckgeschwindigkeit besitzen, mit der der Druck von ganzen Organen real durchführbar ist.

Die erfindungsgemäße Aufgabe wird durch ein neuartiges UV-vernetzendes Droplet - Verfahren gelöst, das aufgrund seiner niedrigviskosen Tinten die hochauflösenden Druckerköpfe von fotorealistischen Kunstdruckern nutzen kann und das zusätzlich durch hochspezifische, räumlich begrenzte Reaktionen im Randbereich der einzelnen Tropfen die Druckauflösung bis auf eine molekulare Ebene erhöht.

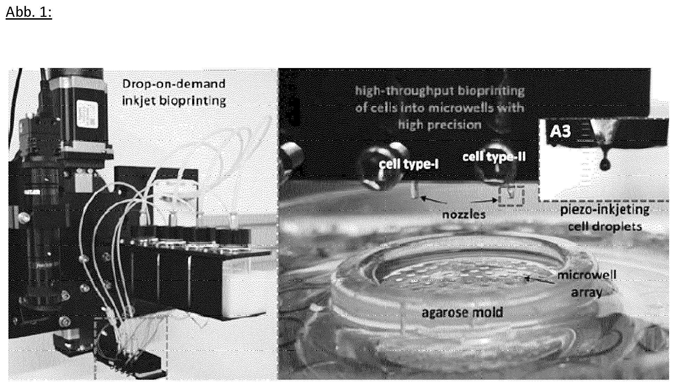

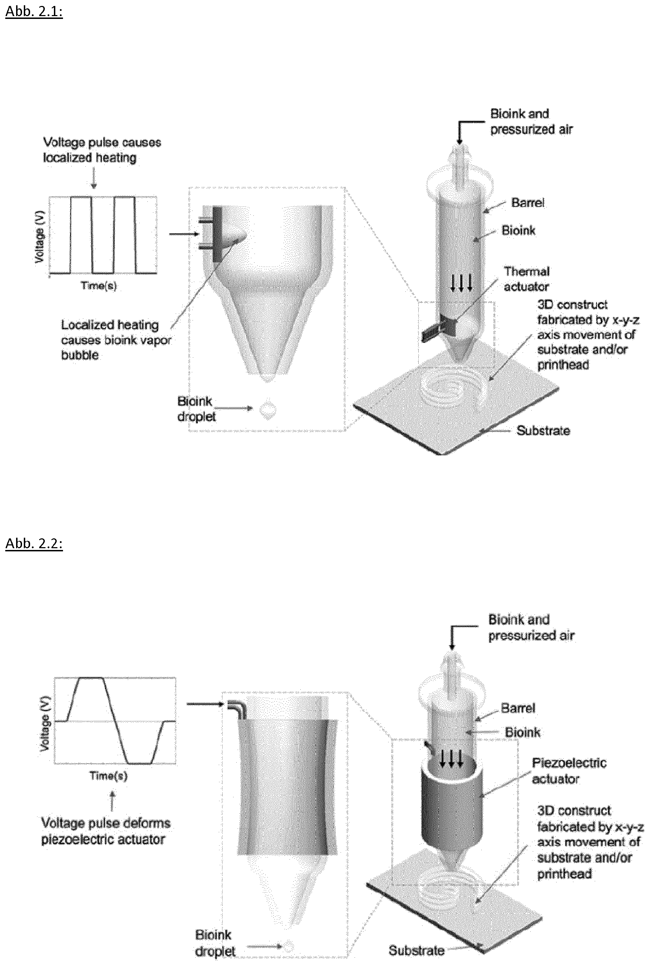

Bisherige Droplet-Verfahren zum Druck von Zellen sind Vorrichtungen, bei denen ähnlich wie durch einen Extruder relativ große Tropfen aus Hydrogel-Tinten und darin befindlichen Zellen vom Druckerkopf abgeschieden werden (thermal oder piezoelektrisch, siehe Abb. 1 und Abb.2). In der Regel existiert für eine Zelltyp-Tinte nur eine einzige Druckerdüse. Diese Düse hat einen relativ großen Durchmesser, da die Viskosität des noch im Solzustand befindlichen Hydrogels trotzdem relativ hoch ist (Abb. 1 und Abb.2). Die dadurch erzeugten Tropfen sind relativ groß und beinhalten eine hohe Anzahl von Zellen (ca. 2000 Zellen pro Tropfen).

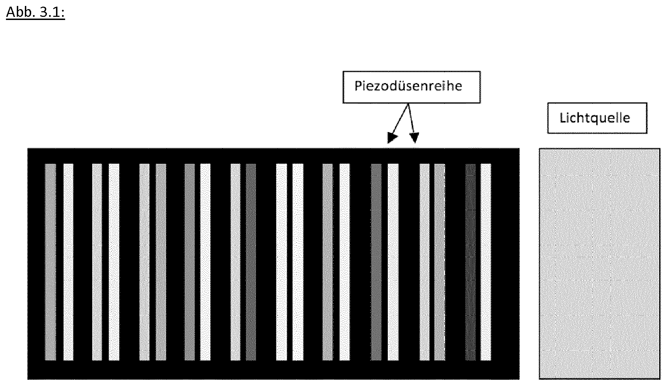

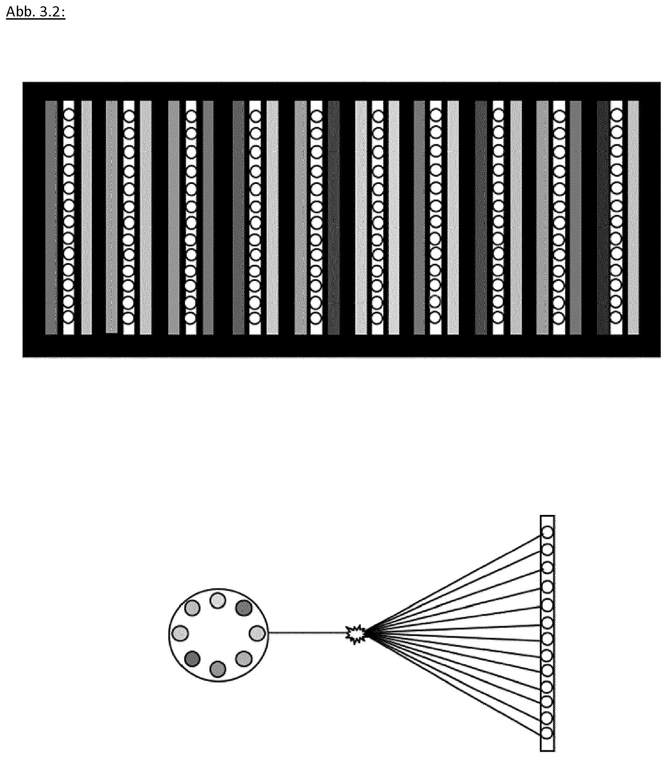

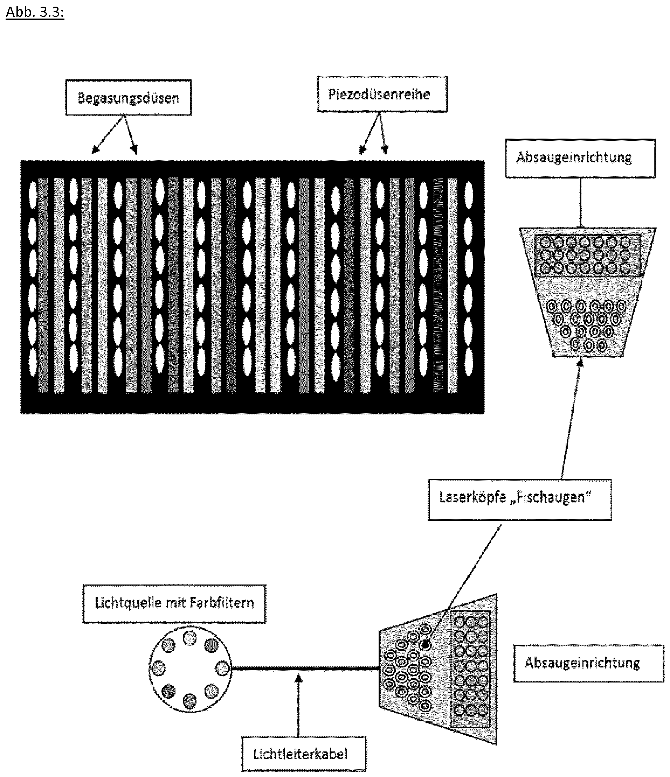

Überraschenderweise hat sich gezeigt, dass durch die Verwendung neu formulierter photosensitiver Zelltinten statt der bisher genutzten Hydrogele die Viskosität der Zelltinten - Tropfen so sehr erniedrigt werden konnte, dass diese von Druckerköpfen aus dem hochauflösenden Kunstdruckbereich verarbeitet werden können. Das war bisher nicht möglich. Dadurch sind Zelltintentropfen von wenigen Pikolitern (mit ca. 20 Zellen pro Tropfen) druckbar (Abb. 3). Mit dieser Druckauflösung kann man bereits Kapillaren drucken, die wesentlich kleiner im Durchmesser sind als die Kapillaren bisheriger Systeme. Zusätzlich stellte sich heraus, dass die Druckauflösung nochmals weiter erhöht werden konnte, indem man die Tinten so gestaltete, dass nur auf den Randbereich der Kapillartintentropfen begrenzt eine Vernetzungsreaktion stattfand. Das restliche Tropfenvolumen hingegen durchläuft diese Vernetzungsreaktion nicht und wird anschließend ausgespült oder auf anderem Wege beseitigt, um anschließend als Kapillarhohlraum zu fungieren. Durch diesen neuartigen Druckvorgang konnte die Druckauflösung bis zu einer Größenordnung auf einer molekularen Ebene bzw. einzelnen Zellen erhöht werden und hat damit die Feinheit von Stereolithographie-Systemen erreicht. Die mit diesem Verfahren druckbaren Kapillaren sind so fein wie die Kapillaren des menschlichen Organismus.

Die auf den Randbereich der Kapillartintentropfen begrenzte Vernetzung kann sowohl durch Tintenbestandteile als auch durch einen gerichteten Laserstrahl erfolgen. Die dabei ablaufenden Reaktionen können physikalischer, chemischer oder biologischer Natur sein (siehe Abschnitt Beispiele).

Während des gesamten Druckprozesses wird das entstehende Gewebe über die kapillaren Strukturen mit Nährmedium durchspült. Soll ein ganzes Organ gedruckt und später in einen Organismus oder Patienten implantiert werden, kann das Organ für einen begrenzten Zeitraum außerhalb des Körpers an den Blutkreislauf angeschlossen werden (siehe Abschnitt Beispiele). In dieser Phase können die physiologischen Werte des Organs überprüft und der optimale Implantationszeitpunkt ermittelt werden.

### Stand der Technik:

In Deutschland warten bis zu 15.000 Patienten auf ein Spenderorgan. Lediglich für weniger als 1/3 von ihnen kann ein passendes Organ gefunden werden. Für Forschung und Pharmazie werden in Deutschland jährlich ca. 3 Mio Tierversuche unternommen, die durch Alternativen überflüssig gemacht werden könnten. Ein weiteres Gebiet für den 3D-Druck von Geweben ist die regenerative Medizin, die weiterhin große Fortschritte macht.

Der 3D-Bioprint kann für diese und andere Anwendungsfelder eine Lösung herbeiführen. Durch ihn werden Zellstrukturen mit 3D-Druckern hergestellt, wobei Zellenebenen in einer additiven Fertigung aufeinandergeschichtet werden, um so komplexe dreidimensionale Strukturen zu bilden. Geeignet für das Biodrucken sind besonders Techniken wie das Fused Deposition Modeling, das Droplet-Verfahren und die Stereolithographie:
Das Fused Deposition Modeling ist ein extrusionsbasiertes Druckverfahren, das Thermoplaste und Verbundwerkstoffe sowie Keramiken und Metalle verarbeitet. Auch Zellen in Alginat- oder Gelatinegelen sind damit druckbar. Nachteilig bei diesem Verfahren ist besonders die geringe Druckauflösung. Das Dropletverfahren verarbeitet polymere Lösungen, kolloidale Suspensionen und Zellsuspensionen. Die Auflösung beim Dropletverfahren ist zwar höher als die des Fused Deposition Modeling, aber nicht ausreichend für den Druck von vaskulären Strukturen. Die Stereolithographie besitzt dagegen durch die Verwendung eines feinen Laserstrahls die höchste Auflösung, die bisher erreichbar ist. Sie verarbeitet eine viskose photoaushärtbare Polymerlösung, die einem gerichteten elektromagnetischen Strahl ausgesetzt wird, um die Lösung räumlich zu vernetzen. Ihr Nachteil liegt darin, dass immer nur ein einziger Zelltyp innerhalb einer Reaktionswanne druckbar ist. Wenn man einen weiteren Zelltyp hinzufügen möchte, muss das Reaktionsbad gewechselt werden.

Im Stand der Technik ist der *Inkjet Biodruck des Marktführers Organovo* beschrieben, der Tintentröpfchen mit jeweils 10.000 bis 30.000 Einzelzellen erzeugt (Quelle: Organovo). Die Firma Organovo nutzt den Biodruck insbesondere zur Herstellung von Lebergewebe. In der Regel werden in das geschädigte Organ 3D-gedruckte Gewebeteile eingepflanzt, die in ihrer Wirkungsweise die bestehende Leber unterstützen und so die Lebensdauer dieses Organs verlängern, bis ein geeigneter Spender gefunden ist.

Beim *Extrusions-Biodruck* werden Biotinten mit Hilfe von mechanischem Druck aus einer kleinen Nadel gespritzt. So wird das gesamte Zellgerüst Schicht für Schicht gedruckt. Dieses Verfahren wird beispielsweise von Biodruckern der Firma *Envisiontec GmbH* genutzt. Die Arbeitsgeschwindigkeit ist relativ hoch, leider ist die Druckauflösung aber sehr niedrig.

Die Stereolithographie wird beispielsweise von den Firmen Cellbricks und TissUse ("organ on a chip") verwendet. Sie liefert die höchste Druckauflösung, da mit Hilfe eines Laserstrahls ein Hydrogel vernetzt wird. Allerdings kann mit diesem Verfahren immer nur ein Zelltyp gedruckt werden. Um das Gewebe aus mehreren Zelltypen zusammen zu setzen, muss das entstehende Gewebe durch verschiedene Reaktionswannen gewechselt werden, wodurch der Prozess verlangsamt wird und das gedruckte Gewebe nur geringe Ausmaße erreicht.

Neben diesen am häufigsten verwendeten Verfahren existieren eine Reihe von Verfahren, die aus Modifizierungen hervorgegangen sind:
Einige Techniken zielen darauf ab, dass sich die sehr feinen Kapillaren (im Durchmesser bis zu 10 µm klein) zwischen den größeren gedruckten Venen und Arterien während der Zellkultur selbst bilden (Yu et al., 2014). Problematisch hierbei ist, dass das gedruckte Gewebe für längere Zeit ausgereift werden muss. Je länger das Gewebe in Zellkultur gehalten wird, desto größer wird die Wahrscheinlichkeit für Artefakte.

Ein anderer Lösungsansatz ist der Druck von schlauchförmigen Strukturen mit einem Polymer, das anschließend wieder depolymerisiert und ausgespült wird und dadurch einen Hohlraum für die Bildung eines vaskulären Systems freigeben soll (Kolesky et al., 2014; Lee et al., 2014a,b). Dieser Lösungsansatz ist aber unzureichend und unterscheidet sich zum hier offenbarten neuartigen Verfahren in zwei Punkten: 1) es handelt sich um einen Schlauch ohne Endothelzellen und feinste Verästelungen, eine längere Inkubation (mit zu erwartenden Artefakten) ist erforderlich und 2) die Druckauflösung ist viel zu niedrig, um ein echtes kapillares System erzeugen zu können.

Ein weiterer Lösungsansatz ist ein Tandem-Druck: zuerst wird mit einem Stereolithographie-System (das eine hohe Auflösung hat aber nur einen einzigen Zelltyp verdrucken kann) das vaskuläre Netzwerk gedruckt. Anschließend wird die gedruckte vaskuläre Struktur in ein anderes 3D-Drucksystem überführt (das keine gute Auflösung hat, aber verschiedene Zelltypen verdrucken kann), in dem die restlichen Gewebezellen gedruckt werden (Yu et al., 2014). Dazu muss die gedruckte vaskuläre Struktur aus dem Reaktionsbehälter des einen 3D-Drucksystems entfernt und in den Reaktionsbehälter des anderen 3D-Drucksystems überführt werden. Besondere Unterschiede zum hier offenbarten Verfahren sind: 1) Es können keine einzelligen Zell-Lagen gedruckt werden, die Strukturen wären bei Wechsel zu fragil und 2) es werden zwei verschiedene Drucker benötigt, beim Wechsel steigt das Kontaminationsrisiko.

Das hier offenbarte neuartige Drucksystem ist ein Dropletverfahren mit photosensitiven Biotinten (elektromagnetische Wellen, z.B. UV, sichtbares Licht, IR) und unterscheidet sich in mindestens 3 Punkten von bisherigen Dropletsystemen, wobei Punkt 2) den Kern des Verfahrens widerspiegelt.
**1)** Es werden neuartige photosensitive Biotinten genutzt, die so niedrigviskos sind, dass sie die feinen Druckerköpfe von photorealistischen Kunstdruckern nicht verstopfen. Bisherige Tintentropfen enthalten ca. 2000 - 3000 Zellen und bestehen aus Hydrogelen (Organovo). Die Hydrogelbestandteile sind der Grund für die relativ großen Tropfen, da ihre Viskosität auch im Solzustand relativ hoch ist. Die im hier offenbarten Verfahren erzeugten Tröpfchen sind sehr klein, niedrigviskos und enthalten ca. 20 Zellen oder weniger. Die gedruckte Zellmasse wird dadurch erreicht, dass die Kunstdruck-Druckerköpfe statt nur einer Düse pro Tintentyp ca. 160 Düsen oder mehr besitzen.
   Gleichzeitig besitzen die Tinten eine Formulierung, die es ermöglicht, unmittelbar zu vernetzen und dadurch ein Verlaufen der Tropfen zu verhindern. Bisher existierende Hydrogele im Dropletverfahren, die photosensitiv waren, nutzen Aushärtungen, die durch eine Lichtquelle außerhalb des Druckerkopfes ausgelöst werden und die über einen längeren Zeitraum von mehreren Minuten andauern. In dem hier offenbarten neuartigen Verfahren befindet sich die Quelle der elektromagnetischen Wellen (z.B. sichtbares Licht, IR, UV) direkt im Druckerkopf und kann sowohl starr als auch gerichtet sein.
**2)** Spezielle Biotinten innerhalb des hier offenbarten neuartigen Drucksystems (z.B. Kapillarttinte) vernetzen nur selektiv begrenzt in den Randbereichen der Tropfen bzw. in den angrenzenden Bereichen zu den Tropfen anderer Tinten. Diese selektive Vernetzung kann entweder durch Bestandteile der Tinte oder durch gerichtete elektromagnetische Strahlung ausgelöst werden. Die Vernetzung kann biologischer, chemischer oder physikalischer Art sein (siehe Abschnitt Beispiele). Die Tropfenbestandteile der speziellen Biotinten, die nicht im Randbereich liegen, werden ausgespült oder anders beseitigt, wodurch der notwendige Hohlraum für die entstehenden Kapillaren gebildet wird.
   Durch diesen Prozess wird es möglich, eine Druckauflösung zu erhalten, bei der nur einzelne Moleküle oder einzelne Zellen gezielt vernetzt werden. Die Eigenschaft, einzelne Zellen zu vernetzen, hatten bisher nur hochauflösende Stereolithographie-Verfahren. Bei der Vernetzung einzelner Moleküle im Randbereich der Tropfen entsteht sogar eine höhere Druckauflösung als im Stereolithographie-Verfahren, das eine maximale Auflösung von ca. 20 nm erreicht.
   Das so erzeugte Druckbild erhält eine neuartige, bisher unerreichte Qualität, bei der
   - einerseits gleichzeitig verschiedene Tintentypen (z.B. Muskelzellen, Nervenzellen, Strukturtinten, Kapillartinten) in photorealistischer Weise gedruckt werden können und bei der
   - andererseits durch die selektiv begrenzte Randvernetzung der Kapillartinten die Druckauflösung so hoch ist, dass einzelne Zell-Lagen (z.B. Endothelzellen) ausgebildet werden.
**3)** Durch die Ausbildung eines belastbaren Blutgefäßsystems bereits während des Drucks ist es möglich, schon während des Druckvorganges das entstehende Gewebe oder Organ ausreichend mit Nährstoffen zu versorgen. Die dazu erforderlichen Anschlüsse an Pumpensysteme werden zunächst auf der Boden- und/oder Dachplatte des Druckers bereitgestellt. Um noch feinere, für jeden Gewebeaufbau individuelle Anschlüsse für Gefäße und Kapillaren zu erzeugen, werden von diesen Anschlüssen aus mit einer Strukturtinte individuelle Anschlüsse gedruckt, an die die Gefäße aus Zellen direkt anschließen. Dabei kann der Übergang zwischen Stukturtinte und Zelltinte über einen Gradienten erfolgen, wodurch das System stabilisiert wird. Bisher gibt es keine Droplet-Technologie, in der ein solches Versorgungssystem für gedruckte Zellen besteht.

Nach erfolgtem Druck kann der Druckerinnenraum fakultativ als Inkubator genutzt und auch außerhalb des Druckers betrieben werden. Beispielsweise können gedruckte Organe im Inkubator für einen begrenzten Zeitraum mit dem Blutkreislauf des Patienten in vitro verbunden werden, um eine Gewebereifung zu erzielen und über die Kontrolle von physiologischen Parametern den richtigen Implantationszeitpunkt zu ermitteln.

### Gegenstand der Erfindung:

Es bedarf eines 3D-Druckverfahrens, das
- innerhalb eines Arbeitsschrittes verschiedene Zelltypen selektiv drucken kann und
- dass die hohe Druckauflösung von Stereolithographie-Verfahren erreicht.
- Zusätzlich soll das Verfahren eine Druckgeschwindigkeit besitzen, mit der der Druck von ganzen Organen real durchführbar ist.

### Die genauen Abläufe des Verfahrens sind:

- Bereitstellung mindestens einer Kapillartinte und einer weiteren Biotinte mit Zellen und Vernetzungsmolekülen in einem Droplet-Drucker;
- Aufbringen mindestens eines Tropfens der Kapillartinte und der Bio-Tinte auf eine Reaktionsebene;
- In-Kontakt-Bringen von elektromagnetischen Wellen mit den Vernetzungsmolekülen in diesen Tropfen auf der Reaktionsebene;
- und Aktivierung der Vernetzungsmoleküle mittels ungerichteter oder gerichteter Bewegungen der elektromagnetischen Wellen in den Tropfen, wodurch vernetzte Strukturen ausgebildet und so vaskuläre Strukturen erhalten werden.
- Bereitstellung mindestens einer Kapillartinte, die nur im Randbereich des Tropfens an der Grenze zu Tropfen anderer Tinten vernetzt
- Ausspülen oder Beseitigen der nicht vernetzten Tropfenbestandteile der Kapillartinte
- Bereitstellen von niedrigviskosen, durch elektromagnetische Wellen vernetzbaren Biotinten, die von den hochauflösenden Druckerköpfen photorealistischer Drucker verarbeitbar sind und die unmittelbar nach Einwirkung der elektromagnetischen Wellen vernetzen
- Bereitstellen eines Versorgungssystems der gedruckten Zellen im Drucker mit Nährmedium

Das erfindungsgemäße 3D-Druckverfahren wird mit einem hochauflösenden Droplet-Drucker und einer Vorrichtung zum Applizieren von elektromagnetischen Wellen bevorzugt einem Laser, einer UV-Lampe oder einer Diode umgesetzt. Die dabei verwendeten Tinten sind niedrigviskose Tinten, die von den hochauflösenden Druckerköpfen photorealistischer Kunstdrucker verarbeitet werden können und die unmittelbar unter dem Einfluss elektromagnetischer Wellen vernetzen, bevorzugt innerhalb einer Sekunde.

Das erfindungsgemäße 3D-Druckverfahren beinhaltet ebenfalls mindestens eine Kapillartinte, die in den Randbereichen der Tropfen bzw. in den angrenzenden Bereichen zu den Tropfen anderer Tinten selektiv vernetzt. Diese selektive Vernetzung erfolgt entweder hervorgerufen durch Tintenbestandteile (der Kapillartinte und/oder der angrenzenden Biotinten) oder durch gerichtete elektromagnetische Wellen, die einzelne Zellen im Randbereich der Kapillartintentropfen vernetzen. Die nicht vernetzten Bereiche der Kapillartinten werden ausgespült oder auf andere Weise beseitigt und bilden den Hohlraum für das Kapillar- und Blutgefäßsystem.

Durch die selektiv auf den Randberiech der Kapillartintentropfen räumlich begrenzte Vernetzung können nicht nur einzelne Zellen, sondern sogar einzelne Moleküle zu einer Vernetzungsreaktion oder anderweitigen Schichtbildung aktiviert werden. Durch diesen Vorgang erreichte Druckauflösung ist noch höher als die von Stereolithographie-Verfahren, die bei ca. 20 nm liegt.

Beispielhafte Reaktionen im Randbereich der Kapillartintentropfen können sein:
- Thiol-en Reaktionen
- Schlüssel-Schloss-Reaktionen z.B. Cycloadditionen
- nucleophile Ringöffnungen
- Selfassembling von Molekülen und Partikeln
- selektive Faktoren an den Zellen der Kapillartinten und/oder Biotinten
- andere Reaktionen mit ähnlicher Wirkung

### Allgemeiner Ablauf des Druckvorgangs:

Der oben beschriebene Droplet-Drucker wird mit Kapillartinte, Biotinten und Strukturtinten beladen. Die Bodenplatte besitzt Anschlüsse für die Versorgung mit Nährmedium und befindet sich über einer Wanne mit Nährmedium, in die die Bodenplatte während des Druckvorganges abgesenkt wird, so dass das bereits gedruckte Gewebe in das Nährmedium eintaucht, wobei die jeweilige Druck-Oberfläche aus dem Medium herausragt.

Um die für den Gewebebereich individuell erforderlichen Anschlüsse für das Blutgefäßsystem zu erzeugen, werden zunächst ausgehend von den Anschlüssen auf der Bodenplatte mit einer Strukturtinte feine Anschlüsse mit geringem Durchmesser gedruckt. Hier erfolgt ein Gradient von Strukturtinte zu Kapillartinte, um einen fließenden Übergang zu schaffen. Nachdem die individuellen Anschlüsse für das Blutgefäßsystem erzeugt wurden, beginnt der Druck mit den Biotinten. Das zu druckende Gewebe wird Schicht für Schicht (entsprechend der Slicer-Software) auf die vorhergehende Schicht aufgetragen. Die neuartigen niedrigviskosen Biotinten vernetzen unmittelbar, so dass es nicht zum Verlaufen der Tropfen kommt. In den Randbereichen zwischen Biotinten und Kapillartinten kommt es zur räumlich begrenzten Vernetzung der Kapillartinten. Nicht vernetzte Bestandteile werden durch das nachströmende Nährmedium ausgespült. Die Spülung kann permanent oder intervallhaft nach einer Reihe von Druckvorgängen erfolgen. Fakultativ kann ausgespültes Material durch Druckluft oder andere Schritte beseitigt werden.

Das so entstandene Gewebe wird nach dem Druck aus dem Drucker entfernt. An dieser Stelle kann man auf verschiedene Weise fortfahren:
a) Die mit Strukturtinte gedruckten Anschlüsse werden von der Bodenplatte abgetrennt. Diese Anschlüsse können zu weiter erfolgenden Anbindungen in natürlichen Geweben oder beim Einbau in Bioreaktoren usw. genutzt werden.
b) Im Druckdesign des Blutgefäßsystems wurde berücksichtigt, dass überwiegende Teile des durch Strukturtinten gedruckten Anschlusses nach Beendigung des Drucks geschlossen werden (z.B. durch einen Laserstrahl) und die Zirkulation des Nährmediums im gedruckten Gewebe oder Organ umorganisiert wird (z.B. Einströmen des Nährmediums durch eine gedruckte Hauptader).

Der Druckerinnenraum kann fakultativ als Inkubator entnommen und auch außerhalb des Druckers betrieben werden. Beispielsweise können gedruckte Organe im Inkubator für einen begrenzten Zeitraum mit dem Blutkreislauf des Patienten in vitro verbunden werden, um eine Gewebereifung zu erzielen und über die Kontrolle von physiologischen Parametern den richtigen Implantationszeitpunkt zu ermitteln.

In einer bevorzugten Ausführungsform der Erfindung liegt die Tropfengröße zwischen einem Femtoliter und einem Mykroliter. Vorteilhafterweise erlaubt diese Tropfengröße die Lösung der erfinderischen Aufgabe überraschend gut.

In einer weiteren bevorzugten Ausführungsform besitzen die Kapillartinten thiolmodifizierte Moleküle, die sie umgebenden Biotinten besitzen allylmodifizierte Moleküle und einen Photoinitiator zur Auslösung der Vernetzungsreaktion. Diese Vernetzungsreaktion findet innerhalb der Biotinten zwischen den allylmodifizierten Molekülen statt. Die thiolmodifizierten Moleküle der Kapillartinten vernetzen nur selektiv im Randbereich mit den allylmodifizierten Molekülen der Biotinten in einer Thiol-en Reaktion. Thiolmodifizierten Moleküle im Inneren des Tropfens vernetzen unter diesen Bedingungen nicht und werden ausgespült.

In einer weiteren bevorzugten Ausführungsform führt eine Kupfer(I)-katalysierte 1,3-dipolare Cycloaddition von Aziden und Alkinen zur Vernetzungsreaktion im Randbereich der Kapillartinten, wobei azidmodifizierte und alkinmodifizierte Moleküle nur im Grenzbereich der Kapillartintentropfen zu Biotinten aufeinander treffen.

In einer weiteren bevorzugten Ausführungsform enthält die Kapillartinte epoxidgruppenmodifizierte Moleküle, die eine nukleophile Ringöffnung mit OH-Gruppen oder NH2-Gruppen von Molekülen aus den Biotinten hervorrufen können oder die durch Komponenten aus den Biotinten eine säurekatalysierte oder basenkatalysierte Ringöffnung durchlaufen.

In einer weiteren bevorzugten Ausführungsform enthalten Kapillartinten und Biotinten Moleküle, die im Grenbereich self-assembling Strukturen ausbilden, beispielsweise über Ionen, Aptamere oder andere zu self assembling neigenden Strukturen.

In einer weiteren bevorzugten Ausführungsform der Erfindung ist vorgesehen, dass die Zellen einen selektiven Faktor enthalten, durch den sie mittels gerichteter Bewegung der elektromagnetischen Wellen innerhalb eines Tropfens selektiv vernetzt werden.

Die neuartigen niedrigviskosen, durch elektromagnetische Wellen vernetzbaren Biotinten und Kapillartinten setzen sich zusammen aus a) dem jeweiligen Zelltyp (fakultativ mit für den Zelltyp selektivem Faktor) und Vernetzungsmolekülen.). Zusätzlich können auch noch Wachstumsfaktoren, Signalmoleküle, Partikel für den Aufbau einer Reizleitung oder andere Moleküle Bestandteile der Tinte sein.

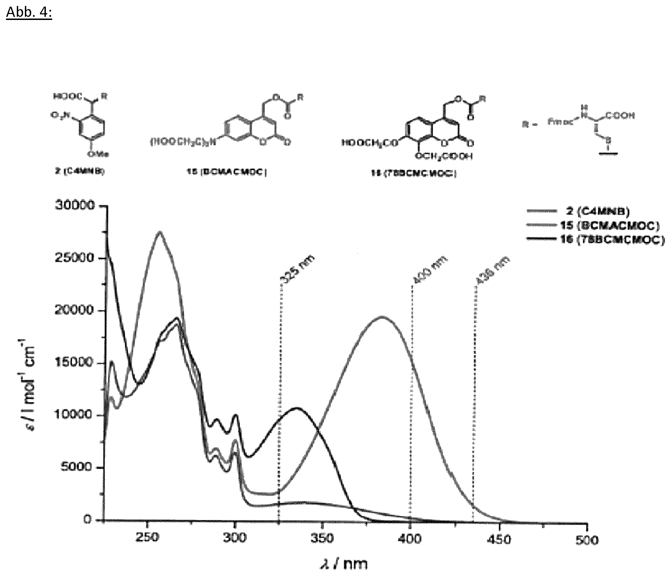

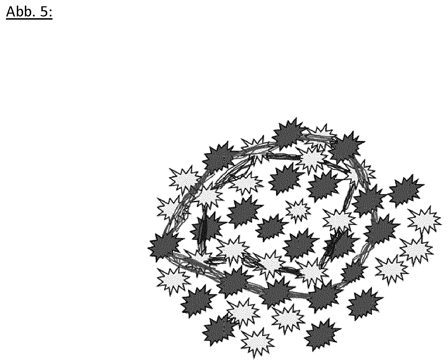

Durch eine selektiv wirkende Energiequelle kann aus einem Tropfen mit einer Mischung verschiedener Zellen eine Vernetzungs- oder Polymerisationsreaktion ausgelöst werden, in der nur der jeweils beabsichtigte Zelltyp getroffen wird. Bevorzugt können die nicht vernetzten Zellen entfernt werden. Beispielsweise kann ein kleiner Tropfen von nur 5 Pikolitern (ca.20 - 200 Zellen) aus einem Gemisch von vaskularen Endothelzellen und Myoblasten bestehen. Aus diesem Gemisch heraus wird in einer bevorzugten Ausführungsform anschließend mit einem nanometergenauen Laserstrahl der Ring einer feinen Kapillare geformt. Durch einen spezifischen Energieeintrag eines Lasers 1 werden im Gemisch bevorzugt nur die Endothelzellen vernetzt, die sich entlang der Innenseite des Ringes befinden. Mit einem anderen spezifischen Energieeintrag eines Lasers 2 werden in dieser bevorzugten Ausführungsform auf der äußeren Seite des Ringes nur die angrenzenden Myoblasten vernetzt (Abb. 4 und 5)

In einer weiteren bevorzugten Ausführungsform ist vorgesehen, dass die nicht vernetzten Zellen im Inneren der vaskularen Struktur mittels Laser entfernt werden.

Bevorzugt werden auch Biotinten bestehend aus einem insbesondere homogenen Zelltyp vernetzt, um beispielsweise größere homogene Gewebebereiche zu drucken.

In einer bevorzugten Ausführungsform werden die zu vernetzenden Zellen in ein sich bildendes Netzwerk eingeschlossen.

In einer weiteren Ausführungsform werden die Zellen selbst untereinander oder mit einem Netzwerk vernetzt. Dazu besitzen sie ein Vernetzungsmolekül, das in der Zellmembran verankert ist.

Durch den vergleichsweise schnellen Druckprozess ist es überraschend möglich, die gedruckten Gewebe relativ zeitnah in einen Inkubator zu überführen, in dem durch Umgebungsfaktoren (z.B. chemische, molekularbiologische, oder durch Stömungsbelastung) eine weitere Reifung ausgelöst wird.

Bevorzugt werden weitere vernetzbare Tinten an bestimmten Positionen verdruckt, die Stützfunktionen für das Gewebe tragen, die Faktoren tragen, die für die Physiologie der Zelle wichtig sind, oder die Wachstumsfaktoren, Marker, Signalmoleküle, Rezeptoren oder weitere Bindungsstellen (Schlüssel-Schloss-Prinzip) tragen.

Der Druckerkopf besitzt ähnlich dem Tintenstrahldrucker für photorealistische Kunstdrucke bevorzugt eine größere Anzahl von Düsen, die auf die jeweilige Rheologie der Tinte angepasst sind. Der Drucker besitzt bevorzugt auch eine oder mehrere Quellen für ungerichtete oder gerichtete elektromagnetische Wellen, durch die die Vernetzungsreaktionen ausgelöst werden.

In einer bevorzugten Ausführungsform wird ein Druckerkopf für photorealistische Kunstdrucke genutzt, der zusätzlich mit einer oder mehreren Quellen für elektromagnetische Wellen modifiziert wurde.

In einer weiteren Ausführungsform wird ein Druckerkopf benutzt, der aus Modulen zusammengesetzt ist, wobei jedes Modul einem Druckerkopf für photorealistische Kunstdrucke mit einer oder mehreren Quellen für elektromagnetische Wellen entspricht.

In einem weiteren Aspekt der Erfindung betrifft diese einen neuartigen Aufbau eines Druckerkopfs (siehe Abb. 3.2 und 3.3) sowie einen neuartigen Druckvorgang, in dem verschiedene Energiequellen selektiv zur Auslösung von verschiedenen Reaktionen genutzt werden (Abb. 3.2 und 3.3).

Abb. 4 und Abb. 5 zeigt schematisiert, wie über verschiedene Photoinitiatoren mit Hilfe von Licht einer spezifischen Wellenlänge bestimmte Zellen miteinander vernetzt werden können. Als Energiequelle können Laser, Dioden oder andere Energiequellen dienen (siehe Abschnitt Beispiele).

In einer bevorzugten Ausführungsform wird das gedruckte Gewebes während des Druckvorgangs ernährt. Die an der Bodenplatte vorhandenen Anschlüsse für das Nährmedium werden individuell auf das zu druckende Gewebe durch Strukturtinten erweitert. Ausgehend von den Anschlüssen an der Bodenplatte werden mit einer Strukturtinte feinere Anschlüsse mit kleineren Durchmessern gedruckt.

In einer bevorzugten Ausführungsform sind in den Piezodüsenreihen eine hohe Anzahl von Piezodüsen pro Reihe untergebracht (ca. 500), die einzeln steuerbar sind und mit den entsprechenden Tinten versorgt werden.

In einer weiteren Ausführungsform ist der Druckerkopf mit einer Bubble-Technologie oder anderen Druckerkopftechnologien für photorealistische Dropletdrucker ausgestattet.

Die Quelle für elektromagnetische Wellen ist in einer Ausführungsform eine ungerichtete Quelle, beispielsweise eine Diode, ein Laser, eine UV-Lampe oder eine andere Quelle.

Die Quelle für elektromagnetischen Wellen ist in einer anderen Ausführungsform eine um 180° schwenkbare Linse. Die Energiequelle ist steuerbar und beweglich, so dass der Laserstrahl jede vorprogrammierte Form abbilden kann. Das Licht wird in einer Ausführungsform in einer externen Lichtquelle generiert und über Lichtleiterkabel zum Austrittsort transportiert.

In einer Ausführungsform ist die Wellenlänge der elektromagnetischen Wellen im UV-Bereich, im sichtbaren Bereich oder im IR-Spektrum.

In einer bevorzugten Ausführungsform ist zur Beseitigung des ausgeschwemmten nicht vernetzten Tintenmaterials eine Absaugvorrichtung oder eine Druckluftvorrichtung vorhanden.

In einer Ausführungsform ist das Vakuum steuerbar und wird nur bei gesenkter Absaugvorrichtung aktiviert. Der Abstand zwischen Absaugvorrichtung und dem zu druckenden Objekt wird durch einen Laser kontrolliert (Abb. 3.3).

In einer weiteren Ausführungsform wird das nicht vernetzte Tintenmaterial durch einen gerichteten Laserstrahl zerstört.

In einer weiteren Ausführungsform werden die nicht vernetzten Zellen, Zelltrümmer oder Tinten durch eine Vorrichtung ausgespült und entweichen in den Reaktionsraum.

In einer Ausführungsform wird über Begasungsdüsen eine Gasatmosphäre oberhalb der Druckebene erzeugt (z.B. Inertgas, CO2, usw.)

In einer Ausführungsform ist die Wellenlänge und die Strahlbreite der Quelle für elektromagnetische Wellen modulierbar. Der Drucker besitzt eine oder mehrere modulierbare Quellen für die gezielt gerichteten elektromagnetischen Strahlen.

In einer weiteren Ausführungsform wird bevorzugt ein Femtosecond Laser eingesetzt.

In einer Ausführungsform wird während des Drucks über das Nährmedium der Gasgehalt (z.B. CO₂/O₂), der pH-Wert und andere wichtige Zellkultur-Parameter geregelt. Zusätzlich wird der Gasaustausch auch über den Gasgehalt im Reaktionsraum reguliert.

In einer Ausführungsform wird das Verfahren als "Bottom Up" oder "up to Bottom" durchgeführt.

In einer Ausführungsform wird das Reaktionsgefäß des Druckers fakultativ als Einweg-Einsatz in den Drucker eingesetzt und kann nach dem Druck entnommen werden. Nach dem Druck ist das Gewebe oder Organ in einem Zustand, in dem es in Kontakt mit dem Patienten gebracht oder in die Zellkultur überführt werden kann. Das Reaktionsgefäß besitzt Anschlüsse für den Zu- und Ablauf von Nährmedium oder Blut zur Ernährung des Gewebes.

In einer Ausführungsform wird das Reaktionsgefäß gekühlt.

In einer Anwendungsform im Patienten wird das Reaktionsgefäß außerhalb des Körpers mit dessen Blutkreislauf verbunden. Durch die bereitgestellten patiententypischen Wachstums- und Differenzierungsfaktoren sowie durch den Blutdruck innerhalb des sich noch differenzierenden vaskulären Systems kommt es zur weiteren Reifung. Während dieser Phase werden fakultativ Proben aus dem System entnommen, um den Reifegrad (Differenzierungsgrad) des Gewebes oder Organs zu überwachen und den optimalen Zeitpunkt für eine Implantation zu ermitteln. Für diese Probennahmen besitzt das Reaktionsgefäß weitere Anschlüsse.

Im Zusammenhang mit der erfindungsgemäßen Lehre ist es vorteilhaft, wenn bestimmte Zelltypen vor dem 3D-Druck mit Molekülen spezifisch hybridisiert werden, um die Zellen in einem Gemisch selektiv ansprechbar zu machen. Die Verbindung besteht hierbei aus einem selektiven Faktor und einem Ankermolekül. Der selektive Faktor soll bevorzugt die Quervernetzung auslösen. Das Ankermolekül dient bevorzugt zum Andocken an die Zellmembran. Der selektive Faktor kann auch direkt ohne Ankermolekül an der Zelloberfläche binden, falls das seine chemischen und physikalischen Eigenschaften zulassen. Ein räumlich gesteuerter, in seiner Wellenlänge und seiner Frequenz speziell gerichteter Energiestrahl aus elektromagnetischen Wellen trifft auf einen selektiven Faktor eines spezifischen Zelltyps und aktiviert diesen beispielsweise über die Abspaltung einer Schutzgruppe, die Aktivierung eines Photoinitiators, die Aktivierung eines Reaktionspartners oder die Aktivierung einer Schlüssel-Schloss-Komponente. Andere Zelltypen werden von dieser Vernetzungsreaktion nicht betroffen. (Abb. 4 und 5, siehe Abschnitt Beispiele).

In einer Ausführungsform besitzt der selektive Faktor als Ankermolekül ein lipophiles Molekül, beispielweise Perfluorcarbone oder Lipide. In einer anderen Ausführungsform besitzt der selektive Faktor als Ankermolekül kationische Molekülbereiche oder Molekülbereiche mit positiven Partialladungen. In einer weiteren Ausführungsfom besitzt der selektive Faktor als Ankermolekül Peptidverbindungen z.B. zellpenetrierende Peptide oder Polynukleotide.

Im Zusammenhang mit der erfindungsgemäßen Lehre werden neuartige Biotinten eingesetzt:
Im Gegensatz zu den bisher genutzten höherviskosen Biotinten herkömmlicher Dropletverfahren mit den darin enthaltenen langkettigen Makromolekülen sind die neuartigen Biotinten des hier offenbarten Verfahrens sehr niedrigviskos und enthalten Photoinitiatoren. Sie enthalten sehr kurzkettige kleinere Vernetzungsmoleküle, die unmittelbar nach dem Kontakt mit elektromagnetischen Wellen vernetzen. In der bevorzugten Ausführungsform besitzen diese Moleküle Allylmodifizierungen oder Thiolmodifizierungen.

### Beispiele:

### 1) Ablauf des 3D-Druckverfahrens:

### 1.1) Modifizierung eines Druckers für Werbedrucke mit UV-vernetzenden Kunststoff-Tinten (6-Farbdrucker) zum Drucker für das neue Droplet-Verfahren:

- der Druckerkopf wurde gegen einen hochauflösenden Epson-Druckerkopf für photorealistische Kunstdrucke ersetzt
- Die UV-Diodenlampe wurde gegen eine Diodenlampe im Bereich von 390 nm ersetzt.
- Es wurde eine absenkbare Bodenplatte installiert, die sich in eine Wanne mit Nährmedium absenken kann. Die Bodenplatte wurde mit Anschlüssen für Nährmedium versehen. An der Unterseite der Anschlüsse wurden Schläuche angebracht, durch die mit Hilfe eines

Perfusors Nährmedium gepumpt wurde. Der vom Perfusor ausgeübte Druck auf das Nährmedium wurde manuell so geregelt, dass sich ein langsam sickernder Flüssigkeitsfluss ausbildete.
- Das RIP-Programm WhiteRip wurde in seinen Funktionen ergänzt und es wurden neue Druckprofile eingerichtet.
- Die UV-vernetzenden Kunststoff-Tinten wurden gegen wasserbasierende UV-vernetzende Tinten ersetzt, die im Wellenlängenbereich von 380 bis 420 nm vernetzen
- Die niedrigviskosen Biotinten wurden in 10 ml Spritzen (ohne Stempel) auf die Tintenreservoir-Anschlüsse des Druckerkopfs platziert, nachdem die ursprünglichen Tintenreservoir-Behälter entfernt wurden.

### 1.2) Ablauf des Druckprozesses:

Der oben beschriebene Droplet-Drucker wird mit einer Kapillartinte, vier Biotinten und einer Strukturtinte beladen. Der Drucker startet mit einer Abfolge von Tropfenabgaben - zunächst Strukturtinten, dann Biotinten und Kapillartinten, um das Druckmuster einer Slice- Ebene zu erzeugen.

Im gleichen Arbeitsschritt bewirkt die UVA 390 nm Diode eine sofortige Vernetzung der Tinten. Mit wachsender Gewebeschicht senkt sich die Bodenplatte in den Reaktionsraum ab, der mit Nährmedium gefüllt ist. Die Druckebene verbleibt oberhalb des Nährmediums.

Um die für den Gewebebereich individuell erforderlichen Anschlüsse für das Blutgefäßsystem zu erzeugen, werden zunächst ausgehend von den Anschlüssen auf der Bodenplatte mit der Strukturtinte feine Anschlüsse mit geringem Durchmesser gedruckt. Die individuellen Anschlüsse aus der Strukturtinte sind in ihrem Durchmesser ca. 100 µm groß und verjüngen sich in Richtung Kapillare. An diese werden zunächst größere Kapillargefäße gedruckt, von denen kleinere Verästelungen abgehen. Für den Druck größerer Kapillargefäße werden mehrere Kapillartintentröpfchen zu einer kreisrunden Struktur gedruckt, die in ihren Randbereichen mit umgebender Biotinte in Kontakt kommt. An den Grenzflächen kommt es zur photosensitiven Vernetzungsreaktion zwischen den allylmodifizierten Molekülen der Biotinten und den thiolmodifizierten Molekülen der Kapillartinte. Dadurch werden Zell-Lagen aus einzelnen Zellschichten aufgebaut. Die nicht vernetzten Kapillartintenbestandteile im Inneren der Kapillarstruktur werden durch das nachsickernde Medium aus den entstehenden Hohlräumen herausgedrückt. Die Spülung der Kapillaren erfolgt in diesem Fall nach 10 Druckschritten. Überschüssiges Material auf der Gewebeoberfläche wird durch ein leichte Druckluftbehandlung beseitigt. Die feinen Kapillarverästelungen werden durch einzelne Kapillartintentropfen erzeugt, die auf die gleiche Weise räumlich begrenzt im Tropfenrandbereich vernetzen.

Das zu druckende Gewebe wird Schicht für Schicht (entsprechend der Slicer-Software) auf die vorhergehende Schicht aufgetragen. Die neuartigen niedrigviskosen Biotinten vernetzen unmittelbar, so dass es nicht zum Verlaufen der Tropfen kommt.

Das gedruckte Gewebe wird aus dem Drucker genommen und in einen Inkubator überführt. Dabei wird die herausnehmbare Bodenplatte mit ihren Anschlüssen für Nährmedium weiter genutzt.

### 1.3) 3D - Druck eines Lebergewebestücks:

Das Lebergewebe besteht hauptsächlich aus dem Leberparenchym, das aus Hepatozyten aufgebaut ist, sowie aus einer Reihe nicht-parenchymatöser Zellen, beispielsweise den Sternzellen, den Pit-Zellen und den Ovalzellen. Diese Zelltypen wurden im Vorfeld generiert und in den entsprechenden Biotinten bereitgestellt. Zusätzlich wurde eine Kapillartinte mit Endothelzellen und eine Strukturtinte bereitgestellt.

Die Informationen über den patientenspezifischen Aufbau der Leber können prinzipiell über eine MRT-Untersuchung erhalten werden. Oft reicht es beim Gewebedruck aber aus, von allgemeinen physiologischen Kenntnissen auszugehen und die zu druckenden Bereiche aus immer wiederkehrenden Modulen zusammen zu setzen, die in ihrem Wesen der natürlichen Vorlage entsprechen, die aber stilisiert sind und in ihrem modularen Aufbau den Druck vereinfachen. In diesem Fall wurden Module gedruckt. Während des Drucks wird der Aufbau der Leber so detailgetreu wie möglich nachgebaut. Trotzdem verbleibt eine Reihe von Strukturen noch unvollendet, da sich deren Zellen weiter differenzieren und sich in eine bestimmte Richtung innerhalb des Zellverbandes ausrichten müssen. Nach Beendigung des Drucks verbleibt das Gewebe auf der Bodenplatte. Der Reaktionsraum mit dem 3D Gewebe oder Organ wird aus dem Drucker herausgenommen und fungiert nun als Inkubator.

Das Blutgefäßsystem wird für den Druck so konzipiert, dass nach Beendigung des Drucks oder der Kultivierung das Gewebe von der Bodenplatte entfernt und der Blutkreislauf den natürlichen Verhältnissen angepasst werden kann. Hierbei wird eine neue, der Natur nachempfundene Zirkulationsroute für das Medium oder Patientenblut eingerichtet, die bereits im Druck angelegt wurde und durch das Verschließen der vom Drucker stammenden Anschlüsse für das Nährmedium ausgeprägt wird. Die bei der Ablösung des Gewebes oder Organs von der Bodenplatte freigelegten Blutgefäße werden durch Lasern oder durch Verkleben (z.B. Fibrinkleber) geschlossen.

### 1.4) Eine erweiterte Variante des Drucker-Aufbaus:

Der Drucker ist aufgebaut aus:
- mehreren Druckerköpfen mit einer Anzahl von Düsen für das Droplet-Verfahren und mit einer oder mehreren räumlich steuerbaren Quellen für elektromagnetische Wellen, die zu einem in Wellenlänge, Intensität und Frequenz modulierbaren Energieeintrag führen
- die modulierbaren elektromagnetischen Wellen dienen der selektiven Aktivierung von Vernetzungsreaktionen
- einer mit Medium gefüllten verschließbaren Reaktionswanne mit Anschlüssen für Mediumwechsel, Probenahmen und nach dem Druck fakultativ geänderter Zufuhrroute für Medium oder Patientenblut, die Reaktionswanne ist aus dem Drucker entnehmbar und kann als Inkubator fungieren
- einer in der Reaktionswanne befindlichen Bodenplatte mit Anschlüssen für das entstehende vaskulare System, durch das kontinuierlich oder in Intervallen Medium strömt und zur Versorgung der gedruckten Gewebe dient
- mehreren Tanks für die erforderlichen Tinten
- verschiedenen Kapillartinten, Biotinten und Strukturtinten:
   - strukturelle Bereiche (z.B. Stützstrukturen, extrazellulärer Matrix oder andere nicht zelluläre Bestandteile, beispielsweise Polyhydroxyalkanoate, Fibrinogene, Collagenen, Myeline, Glycosamine, Glycolipide, Peptide, Zuckermoleküle, Fettsäuren, Schlüssel-Schloss-Moleküle, organische und anorganische Moleküle für die Reizleitung usw.)
   - Eigenschaftsmoleküle (z.B. Moleküle verschiedener physiologischer Eigenschaften, beispielsweise Wachstumsfaktoren, Transkriptionsfaktoren, Rezeptormoleküle, Signalmolekülen, RNA, DNA, Differenzierungsmoleküle usw.)
   - homogene Zelltypen (z.B. Myozyten, Leberzellen, Neuronen, usw.)
   - Gemische aus heterogenen Zelltypen (z.B. Endothelzellen, Muskelzellen) für den Druck des vaskularen Systems
- Die Bodenplatte ist während des Drucks auf - und abfahrbar, fakultativ kann stattdessen der Druckerkopf auf- und abfahrbar sein oder beides zusammen.

### 3) Beispiel einer Reifung des gedruckten Gewebes im Inkubator

Der Inkubator dient der Ausdifferenzierung des gedruckten Gewebes oder Organs. Nach dem Drucken ist zwar schon ein Gewebekomplex mit hoher Zelldichte entstanden, die Zellen haben aber noch nicht ihre endgültige Gewebestruktur und Differenzierung erlangt. Das Gewebe oder Organ wird direkt in eine Kammer gedruckt, die später als Inkubator dient. Fakultativ kann das Gewebe oder das Organ auch in einen weiteren Inkubator überführt werden. Der Inkubator liefert wichtige Differenzierungsparameter, die für die Ausdifferenzierung notwendig sind, beispielsweise chemische oder molekularbiologische Stoffe oder Strömungsparameter.

Der Inkubator kann fakultativ mit Nährmedium betrieben werden und zu einem späteren Zeitpunkt außerhalb des Körpers mit dem Blutkreislauf des Patienten verbunden werden oder der Inkubator wird gleich nach dem Druck mit dem Blutkreislauf des Patienten verbunden. Beim Kontakt mit Patientenblut gelangen notwendige Differenzierungs- und Wachstumsfaktoren zu den gedruckten Strukturen und die Zellen können an den Körper adaptieren.

### 4) Aufbau des Druckerkopfs:

Der Druckerkopf besteht aus einem *ersten Teilbereich* für den Droplet - Druck der Biotinten, Kapillartinten und Strukturtinten. Das Droplet-Verfahren läuft in der Regel über über Piezo-Düsen oder Bubble-Düsen. Der Druckvorgang ist im Ablauf und der erzeugten Anordnung der Tropfen ähnlich dem Cyan/Magenta/Yellow/Schwarz/Grau/Hellgrau/Light Magenta/Light Cyan - System von Fotodruckern. Anstelle der Farbtinten steht die erforderliche Anzahl von Biotinten, Kapillartinten und Strukturtinten.

In einem *zweiten Teilbereich* besitzt der Druckerkopf eine oder mehrere ungerichtete oder gerichtete Quellen für elektromagnetische Wellen. Die gerichteten Quellen erzeugen in ihrer Gesamtheit eine selektive Energiequelle, die bezüglich Wellenlänge, Intensität und Frequenz modulierbar ist. In der Regel dienen als Quellen für elektromagnetische Wellen UV-Lampen, Leuchtdioden, Laser oder Screens. In der Regel verwendete elektromagnetische Wellen sind ultraviolettes Licht, sichtbares Licht und Wellenlängen im Infrarotspektrum.

Die Funktionseinheiten des Druckerkopfs für das Dropletverfahren und für das Verfahren zur Bereitstellung der elektromagnetischen Wellen können auch voneinander getrennt angeordnet sein. Ebenfalls kann der Druckerkopf aus einer Reihe von Modulen solcher Einheiten aufgebaut sein.

### 5) Aufbau der Reaktionswanne mit Bodenplatte, Zufuhr von Medium:

In der Reaktionswanne des Druckers befindet sich eine auf- und abfahrbare Bodenplatte mit einer Anzahl von Anschlüssen für die Durchströmung der gedruckten vaskularen Strukturen mit Medium.

Außerhalb der Reaktionswanne sind die Anschlüsse mit einem externen System zur Aufrechterhaltung von bestimmten Zellkulturparametern verbunden. Dazu gehören u.a. Temperatur, pH-Wert, CO₂-Gehalt, O₂-Geahlt, Nährstoffe, Wachstumsfaktoren usw.. Da auch die nicht vernetzten Zellen innerhalb der gedruckten vaskularen Strukturen in Form von Zelltrümmern oder als ganze Zellen ausgespült werden, muss das in die Reaktionswanne abfließende Medium in seinen Parametern kontrolliert und gegebenenfalls gereinigt oder getauscht werden.

Während des Drucks des vaskularisierten Gewebes wird Medium durch die Anschlüsse in die darauf aufgedruckten vaskularen Strukturen geleitet. Dies dient einerseits der Ernährung des entstehenden Gewebes und andererseits dem Freispülen der Hohlstrukturen des vaskularen Systems. Das Medium tritt durch die Anschlüsse der Bodenplatte in die vaskularen Strukturen, durchströmt das soweit gedruckte vaskularisierte Gewebe und sickert oberhalb an der dem Druckerkopf zugewandten Seite an den noch offenen Hohlräumen des vaskularen Systems wieder aus. Von dort fließt das Medium entweder passiv zurück in die Reaktionswanne oder wird während des Druckprozessen aktiv von der Druckoberfläche entfernt (z.B. abspülen bzw. absaugen).

Die Bodenplatte senkt sich während des Druckvorganges immer weiter in den Reaktionsraum, so dass das gedruckte Gewebe mit dem Medium in der Reaktionswanne umspült ist. Ca. 1 - 5 mm des obersten Bereichs des Gewebes ragt aus dem Medium hervor. Da die Thiol-en - Reaktionen relativ O₂-unempfindlich ablaufen, ist es in der Regel nicht erforderlich, die luftgefüllten Bereiche der Reaktionswanne mit Inertgas (z.B. Stickstoff, Argon) zu füllen.

### 6) Bereitstellung der notwendigen digitalen Informationen für die 3D-Drucker - Software

Die digitalen Daten über den zellulären Aufbau von Geweben und Organen werden aus medizinischen Bildgebungsverfahren erhalten und zu 3D-Druckdaten programmiert. Zu diesen Verfahren gehören beispielsweise das MRT - Verfahren oder das neue "DISCO-Transparenz" - Verfahren. Die Verfahren zur Ermittlung dieser Daten sind dem Fachmann bekannt.

### 7) Neuartige Druckersoftware:

Die Druckersoftware verarbeitet die Informationen über die zu druckenden Gewebereiche und gibt diese zur Ausführung an den Druckerkopf und die Reaktionswanne weiter. Im Gegensatz zu herkömmlichen 3D-Drucker-Programmierungen werden hier sowohl die Droplet-Technologie (Druckmuster der spezifischen Tinten und Tropfengröße) als auch im Fall einer gerichteten Quelle für elektromagnetische Wellen eine modulierte Lasertechnologie (geforderte Wellenlängen, Energie-Intensitäten und Frequenzen, räumliche Aussteuerung) miteinander kombiniert und aufeinander abgestimmt. Eine zentrale Rolle dabei spielt die räumliche und zeitliche Koordinierung der Tropfenabgabe spezifischer Tintentypen in die Reaktionswanne mit der zu deren Vernetzung notwendigen räumlichen und zeitlichen Bereitstellung elektromagnetischer Wellen in einer tintenspezifischen Wellenlänge, Frequenz und Intensität. Im Mittelpunkt steht die selektive Vernetzung von Endothelzellen und von Muskelzellen innerhalb eines Tintentropfens aus einem Zellgemisch heraus zu vaskularen Strukturen (Abschnitt "Verwendete Schutzgruppen und Photoinitiator")

### 8) Reifung eines gedruckten Gewebes und Organs bis zur Transplantation:

Der Inkubator mit Gewebe oder Organ besitzt Anschlüsse für die ex vivo Anknüpfung an den Blutkreislauf des Patienten oder an ein Medium lieferndes System. Durch eine Inkubation ausgehend von einigen Stunden bis einigen Tagen wird das Zellgefüge des 3D Drucks weiter ausdifferenziert. Besonders durch die Verbindung des Gefäßkreislaufs des Drucks mit dem Blutkreislauf des Patienten werden wichtige Entwicklungsfaktoren an die Zellen geliefert. Die Endothelzellen und Muskelzellen der gedruckten vaskularen Strukturen benötigen außerdem zur Differenzierung einen spezifischen Gefäßinnendruck durch das Blut oder das Medium und eine spezifische Strömungsgeschwindigkeit.

Während der Reifungsphase ist es möglich, über Anschlüsse im Inkubator spezifische physiologische Werte zu überprüfen und damit die Reifung des Organs oder Gewebes zu überwachen. Wenn die physiologischen Werte des gedruckten Organs oder Gewebes in einem bestimmten Rahmen die physiologischen Werte eines natürlichen Organs erreichen, kann die Transplantation in den Körper des Patienten erfolgen.

### 9) Aufbau der neuartigen niedrigviskosen Tinten:

Der prinzipielle Aufbau der Tinten besteht aus niedrigviskosen, kurzkettigen Vernetzungsmolekülen, aus Photoinitiatoren und aus Zellen. Die kurzkettigen Vernetzungsmoleküle besitzen mindestens eine in der Regel 2 oder mehr funktionelle Gruppen, die in der Regel aus Allylgruppen oder Thiolgruppen bestehen und Polymerisations- oder Additionsreaktionen durchlaufen.

Häufig verwendete Vernetzungsmoleküle sind methacrylatmodifizierte und thiolmodifizierte Moleküle, z.B. methacrylatmodifizierte oder thiolmodifizierte Peptidketten, Polyhydroxyalkanoate, oder Hyaluronsäuren. Die Biotinten und Strukturtinten sind entweder überwiegend aus methacrylatmodifizierten Molekülen (radikalische Polymersierung oder Addition) aufgebaut oder auch aus einer Mischung von methacrylatmodifizierten Molekülen mit thiolmodifizierten Molekülen (Thiol-en Reaktionen). Die Kapillartinten hingegen sind in der Regel aus reinen thiolmodifizierten Vernetzungsmolekülen aufgebaut, die nur an den Randbereichen des Tropfens in Wechselwirkung mit den allylmodifizierten Vernetzungsmolekülen der Biotinten und Strukturtinten vernetzen.

In der Regel werden Photoinitiatoren verwendet, die entweder im Bereich von ca. 380 nm arbeiten oder mit Photoinitiatoren, die im Infrarotbereich liegen. Bei Photoinitiatoren im Infrarot-Bereich handelt es sich um Stereo-Energiequellen ähnlich der Multiphotonenstereolithographie (nichtlineare Mehr-Photonen-Absorption), bei der verschiede Energiequellen überlagert werden müssen, um eine Reaktion auszulösen.

Beim Druck von Zellen mit selektiven Faktoren (z.B. spaltbare Schutzgruppen für Thiolgruppen) werden zusätzliche Photoinitatoren unterhalb von 380 nm verwendet.

Beispiele für verwendete Photoinitiatoren sind DAROCUR 1173, IRGACURE 2959 UND 369, *(Ru(phen)₃*/*lod*/*(TMS)₃Si-H PIS) und Photoinitiator 183 aus "*P. Xiao et al./Progress in Polymer Science 41 (2015) 32 - 66, S. 46". Photoinitiatoren für IR-Anregungen sind *H-Nu-IR 780* und *H-Nu-IR* 815. Es hat sich herausgestellt, dass eine Reihe von Photoinitiatoren aus dem Bereich des sichtbaren Lichts ebenfalls durch Multiphotonen aktivierbar sind.

### 10) Bereitstellen von Zellen, die zum Druck erforderlich sind:

Zellen für das hier offengelegte neuartige 3D-Druckverfahren können auf verschiedene Weise bereitgestellt werden. Der Ursprung der Zellen hängt dabei vom beabsichtigten Verwendungszweck des damit gedruckten Gewebes oder Organs ab.

Der einfachste Weg, Zellen zu generieren, ist die Verwendung von käuflichen Zell-Linien. Mit solchen Zellen lassen sich Druckparameter und Kultivierungsparameter optimieren, da man für diese Tests nicht die relativ kostspieligen induzierten Stammzellen (iPS) oder aus Organen und Geweben isolierte Zellen verwenden muss.

Eine weitere Quelle für Zellen ist die Isolierung bestimmter Zelltypen aus Organen und Geweben. Neben tierischen Zellen für die Forschung wird dieser Prozess auch schon vielfältig in der regenerativen Medizin eingesetzt, beispielsweise die Isolierung von Bandscheiben-Knorpelzellen aus Prolaps-Material von Bandscheibenoperationen. Das Prolaps-Material wird aufgelöst und die Zellen werden daraus isoliert, vermehrt und u.a. auch im 3D-Druck von Geweben eingesetzt.

Die wohl anspruchsvollste Quelle für Zellen ist die Generierung von induzierten Stammzellen (iPS). Hierfür existieren Vorgehensweisen verschiedener etablierter Arbeitsgruppen. iPS-Zellen sind für den Druck menschlicher Gewebe und Organe erforderlich, die anschließend in den menschlichen Körper implantiert werden sollen. Aber auch in der Zellkultur als Testmaterial für individualisierte Medikamente sind diese Zellen und die daraus gedruckten Strukturen einsetzbar. iPS-Zellen werden in der Regel aus Hautzellen des Spenders gezüchtet. Die Hautzellen werden zu iPS-Zellen defifferenziert, vermehrt und in die zum Druck erforderlichen Zelltypen differenziert. Diese Vorgänge sind für eine breite Anwendung noch wenig etabliert, stellen aber die Zukunft bei der Herstellung von menschlichen autologen Implantaten für Organe und Gewebe dar.

Weitere Quellen für Zellmaterial sind für spezifische Zwecke gezüchtete Zelllinien, die in der Regel auf verschiedene Weise modifiziert wurden (z.B. auf DNA-Basis, auf RNA- oder Proteinbasis).

### 11) Beispielhafte Tinten für den Druck mit Zellen, die einen selektiven Faktor enthalten:

Eine Möglichkeit, Kapillartinten nur im Randbereich zu vernetzen, ist der Einsatz von Zellgemischen aus Endothelzellen und Muskelzellen, die innerhalb eines Tropfens selektiv miteinander vernetzt werden. Um von einem gerichteten Laserstrahl selektiv aktivierbar zu sein, benötigen diese Zelltypen einen spezifischen selektiven Faktor. Dieser selektive Faktor ist beispielsweise eine spaltbare Schutzgruppe auf einer funktionellen Thiolgruppe, die sich auf der Zellmembran des betreffenden Zelltyps befindet. Durch die Verwendung verschiedener Schutzgruppen, die sich bei unterschiedlichen Wellenlängen spalten lassen, können die verschiedenen Zelltypen in ihrer Vernetzungsreaktion voneinander getrennt werden. In der Regel besteht die Kapillartinte bei dieser Anwendung aus thiolmodifizierten Vernetzungsmolekülen mit Schutzgruppe, die an der Zellmembran verankert sind und aus freien thiolmodifizierten, geschützten Molekülen in der Tinte. Zusätzlich befinden sich allylmodifizierten Moleküle in der Tinte, um eine Thiol-en - Vernetzungsreaktion zu ermöglichen.

### 11.1) Aufbau der Tinten und selektiver Faktor:

Die lichtabhängige Thiol-en - Reaktion erfolgt innerhalb des vom Laser festgelegten räumlichen Vernetzungsbereichs selektiv über selektive Faktoren. Diese selektiven Faktoren bestehen beispielsweise aus lichtspaltbaren Schutzgruppen, die die Thiolgruppen blockieren. Aufgrund ihres unterschiedlichen molekularen Aufbaus sind diese Schutzgruppen durch verschiedene Wellenlängen des Lichts spaltbar. Es werden solche Schutzgruppen auswählt, die sich im Bereich ihres Anregungsspektrums nicht überschneiden.

Um in einem vom Laser getroffenen Bereich nur bestimmte Zelltypen aus einem Gemisch heraus selektiv zu vernetzen, andere aber nicht, müssen an den betreffenden Zellen die Schutzgruppen an den Thiolgruppen gespalten werden. Somit sind nur diese Zellen und diese Vernetzungsmoleküle einer Thiol-en - Reaktion zugänglich. Anschließend wird in diesem Beispiel die Thiol-en - Vernetzungsreaktion durch einen Photoinitiator gestartet, dessen Anregungsspektrum oberhalb der Wellenlänge aller verwendeten Schutzgruppen liegt, um diese nicht versehentlich zu spalten (Beispiele siehe unten).

### 11.2) Druck des vaskularen Systems mit Zellen die einen selektiven Faktor enthalten:

Da der Durchmesser der feinen Kapillaren zwischen den Arterien und Venen nur wenige Mikrometer beträgt, die Tropfen im Droplet-Verfahren aber größer sind, wird der innere einlagige Ring aus Endothelzellen aus einem Tropfen einer Zellmischung heraus selektiv vernetzt. Ebenso wird der diesen Ring umgebende Ring aus Muskelzellen aus dem Zellgemisch heraus selektiv vernetzt. Hierfür tragen die Endothel- bzw. die Muskelzellen als selektiven Faktor jeweils zu unterschiedlichen Wellenlängen spaltbare Schutzgruppen (siehe Beispiele unten).

Nach einer selektiven Spaltung der Schutzgruppen am entsprechenden Zelltyp kann bei den entschützten Zellen die Thiol-en - Reaktion ablaufen. So werden zunächst durch einen Laserstrahl 1 im Tropfen ca. 5 - 7 Endothelzellen und die dazwischen liegenden geschützen thiolmodifizierten Vernetzungsmoleküle zu einer ringförmigen Struktur entschützt und anschließend durch einen Laserstrahl 2 untereinander ringförmig vernetzt. Der Laserstrahl 2 dient der Anregung eines Photoinitiators und liegt oberhalb der Wellenlänge von Laserstrahl 1 und 3.

Dadurch entsteht ein einlagiger Endothelring mit einem Innendurchmesser von ca. 10 Mikrometern, dessen innere Fläche aus nicht vernetzten, geblockten Zellen besteht. Anschließend werden durch einen Laserstrahl 3 die dem Endothelring benachbarten äußeren Muskelzellen und geschützten thiolmodifizierten Vernetzungsmoleküle entschützt und mit einem Laserstrahl 2 untereinander zu einem Außenring vernetzt. Während die Laserstrahlen 1 und 3 bei unterschiedlichen Wellenlängen die entsprechenden Schutzgruppen spalten, führt Laserstrahl 2 zur Aktivierung eines Photoinitiators, der die Thiol-en - Reaktionen startet.

Die unvernetzten Zellen im inneren der Kapillaren werden durch einen Laserstrahl höherer Energie (Laserstrahl 4) zerstört und die entstehenden Zelltrümmer vom einströmenden Medium ausgespült.

### 11.3) Verwendete Schutzgruppen und Photoinitiatoren bei Tinten mit selektiven Faktoren:

Während für die Vernetzung von Tinten mit homogenen Zelltypen der Reaktionstyp frei gewählt werden kann und unselektiv abläuft, besitzen die verschiedenen Zelltypen mit heterogenen Tinten Thiol-en - Reaktionspartner mit einer spezifischen Schutzgruppe, die zur entsprechenden Wellenlänge spaltbar ist. Dabei dürfen sich die Kurven der notwendigen Aktivierungsenergien nicht überlappen (Abb. 4).

Für die Vernetzung der Endothelzellen bzw. der diese umgebenden Muskelzellen wurden folgende UVspaltbare Schutzgruppen verwendet:
*325 nm: (7,8-Bis(carboxymethoxy)-cumarin-4yl)methoxycarbonyl für Muskelzellen*
*400 nm: α Carboxy-4methoxy-2-nitrobenzyl für Endothelzellen*
   (Quelle: Wellenlängenselektiv abspaltbare photolabile Schutzgruppen für Thiole, Nico Kotzur, 2009)
Zunächst wird die Schutzgruppe mit der höheren erforderlichen Wellenlänge abgespalten, um die andere Schutzgruppe nicht versehentlich zu aktivieren. Für die eigentliche Thiol-en - Reaktion wird ein Photoinitiator *(Ru(phen)₃*/*lod*/*(TMS)₃Si-H PIS)* verwendet, dessen Anregungsspektrum bei *532 nm* liegt, um die darunter liegenden Schutzgruppen nicht versehentlich zu spalten. Ebenfalls einsetzbar ist der *Photoinitiator 183 (*P. Xiao et al./Progress in Polymer Science 41 (2015) 32 - 66, S. 46), der bei *473 nm* angeregt wird.

In einem ersten Schritt wird die Schutzgruppe an den Thiolgruppen der Endothelzellen bei 400 nm abgespalten (Laserstrahl 1). In diesem Bereich sind die Thiolgruppen der Muskelzellen noch geschützt. Danach wird der Photoinitiator bei 532 nm aktiviert und eine Thiol-en - Reaktion an den entschützten Gruppen ausgelöst (Laserstrahl 2). Durch die räumliche Führung der beiden nacheinander folgenden Laserstrahlen (erst 400 nm, dann 532 nm) werden in der Regel 5 bis 12 Endothelzellen ringförmig miteinander vernetzt.

In einem zweiten Schritt werden bei 325 nm die Schutzgruppen an den Thiolgruppen der Muskelzellen abgespalten Laserstrahl 3). Da die räumliche Führung der Laserstrahlen für den Bereich der Endothelzellen (400 nm und 532 nm) den Bereich des die Endothelzellen umgebenden Muskelringes nicht trifft, liegen dort die Endothelzellen der Zellmischung noch mit Schutzgruppen vor und können an der Thiol-en - Reaktion der entschützten Thiolgruppen der Muskelzellen nicht teilnehmen. Anschließend erfolgt durch die Aktivierung des Photoinitiators bei 532 nm eine Vernetzung der im Ring befindlichen Muskelzellen (Laserstrahl 2).

Auf diese Weise kann sichergestellt werden, dass überwiegend nur der gewünschte Zelltyp in die entsprechende Struktur eingebaut wird.

Für weitere benötigte selektive Faktoren stehen zusätzliche Schutzgruppen für Thiolgruppen zur Verfügung (Abb. 4), z.B. die bei 436 nm spaltbare Gruppe ((7Bis(carboxymethyl)amino)-cumarin-4-yl)methoxycarbonyl). Der Vernetzungsablauf erfolgt analog wie oben beschrieben. (Quelle: Wellenlängenselektiv abspaltbare photolabile Schutzgruppen für Thiole, Nico Kotzur, 2009)

Die nicht vernetzen Zellen im Inneren der Kapillaren werden durch einen Laserstrahl 4 höherer Energie zerstört und die Trümmer vom Medium ausgespült.

### 12) Beispielhafte Herstellung von autologen Patientenzellen mit einem selektiven Faktor

Die vom Patienten entnommenen Zellen (z.B. Hautzellen) werden zu induzierten Stammzellen (iPS) umgewandelt, vermehrt, in einzelne Fraktionen gesplittet und danach in die erforderlichen Zelltypen differenziert. Diese Vorbereitung erfolgt unabhängig vom Druckprozess und wird hier nur beispielhaft erwähnt, um den Weg zu echten 3D-Organen zu beschreiben. Dieser Vorgang kann Wochen bis Monate dauern, je nachdem Komplexität des zu druckenden Organs. Nach Produktion der erforderlichen Zelllinien können diese eingefroren und gelagert werden, bis der Druck erfolgen soll. Man kann eingefrorene Zellen auftauen, mit dem selektiven Faktor hybridisieren und drucken. Schonender für die Zellen ist eine Inkubationszeit nach dem Auftauen in der Zellkultur für 24 Stunden und eine anschließende Hybridisierung mit ihrem spezifischen selektiven Faktor.

In der Regel ist der selektive Faktor mit einem Ankermolekül für die Verankerung in die Zellmembran verbunden. Die verwendeten Ankermoleküle sind in der Regel universell für alle Zelltypen einsetzbar. Überraschenderweise hat sich gezeigt, dass perfluorierte Ankermoleküle am wirksamsten sind. Erfahrungsgemäß binden diese Moleküle innerhalb von 2 Minuten an 100% der beteiligten Zellen. Als Ankermoleküle können aber auch lipophile Moleküle, Peptide, Ionen oder Antikörper fungieren.

### 13) Vorbereitung der Zellen mit selektivem Faktor zum Druck:

Kurz vor dem Druck werden die Zellen des Kapillarsystems mit ihrem spezifischen selektiven Faktor hybridisiert: Die verwendeten Endothelzellen und Muskelzellen werden zunächst getrennt in der Zellkultur auf den Druck vorbereitet und mit ihrem jeweiligen selektiven Faktor hybridisiert. Anschließend werden sie in den Kapillartinten zusammengeführt.

### Abbildungen:

Abb. 1: Droplet-Bioprinter mit nur einer Düse pro Zelltyp, beispielsweise genutzt vom Marktführer Organovo; Quelle: 3D Bioprinting - Fundamentals, Prinziples and Applications, Ibrahim T. Ozbolat, Elsevier
Abb. 2.1: Der Mechanismus eines thermischen Systems, "drop-on-demand" Bioprint. Quelle: 3D Bioprinting - Fundamentals, Prinziples and Applications, Ibrahim T. Ozbolat, Elsevie
Abb. 2.2: Der Mechanismus eines piezoelektrischen Systems, "drop-on-demand" Bioprint. Quelle: 3D Bioprinting - Fundamentals, Prinziples and Applications, Ibrahim T. Ozbolat, Elsevier
3.1: Beispielhafter Aufbau eines photorealistischen hochauflösenden Dropletdruckerkopfes mit einer ungerichteten Quelle für elektromagnetische Wellen
   In den Piezodüsenreihen sind eine hohe Anzahl (ca. 500) von Piezodüsen pro Reihe untergebracht, die einzeln steuerbar sind und über Leitungen mit der Tinte und den Zellen versorgt werden. Jedes Piezoelement, kann eine Tintenmenge von 2 - 6 Pikolitern verdrucken.
Abb. 3.2: Beispielhafter Aufbau eines photorealistischen hochauflösenden Dropletdruckerkopfes mit modulierbarer Quelle für elektromagnetische Wellen
   Die Quelle für elektromagnetische Wellen ist in einem separaten Modul des Druckkopf angebracht und erzeugt Energie unterschiedlicher Wellenlänge entsprechend eines spezifischen Laserstrahls oder einer spezifischen Diode.
Abb. 3.3: Beispielhafter Aufbau eines photorealistischen hochauflösenden Dropletdruckerkopfes mit erweiterten Funktionen wie modulierbare Quelle für elektromagnetische Wellen (Lichtquelle mit Farbfiltern) und gerichteten Quellen für elektromagnetische Wellen (Laserköpfe "Fischaugen"), sowie einer Absaugvorrichtung für nicht vernetzte oder gebundene Zellbestandteile der Kapillartinten und mit Begasungsdüsen zur Herstellung einer Gasatmosphäre oberhalb der Gewebeoberfläche
Abb. 4: spaltbare Schutzgruppen für Thiolgruppen zu den Wellenlängen von 325nm, 400 nm und 436 nm (Quelle: Wellenlängenselektiv abspaltbare photolabile Schutzgruppen für Thiole, Nico Kotzur, 2009)
Abb. 5: Variante der Kapillartinten: Zellen mit selektivem Faktor. Helle Zellen zeigen Endothelzellen, dunkle Zellen zeigen Muskelzellen. Beide Zelltypen liegen als Gemisch in einem Kapillartintentropfen vor und können selektiv zur Vernetzungsreaktion aktiviert werden.

## Patentansprüche

1. **3D-Druckverfahren** zur Herstellung von vaskulären Strukturen aufweisenden Geweben und Organen mittels
**(a)** eines Droplet-Druckers für photorealistische hochauflösende Drucke und
**(b)** einer Vorrichtung zum Applizieren elektromagnetischer Wellen umfassend die Schritte:
- Bereitstellung mindestens einer Biotinte und einer Kapillartinte mit Zellen und Vernetzungsmolekülen in dem Droplet-Drucker
- Aufbringen mindestens eines Tropfens der Biotinte und der Kapillartinte auf eine Reaktionsebene
- in-Kontakt-bringen der elektromagnetischen Wellen mit den Vernetzungsmolekülen in diesen Tropfen auf der Reaktionsebene
- und Aktivierung der Vernetzungsmoleküle mittels ungerichteter oder gerichteter Bewegungen der elektromagnetischen Wellen in den Tropfen, wodurch vernetzte Strukturen ausgebildet und so vaskuläre Strukturen erhalten werden.

2. Verfahren nach Anspruch 1,
**dadurch gekennzeichnet, dass**
die Tropfengröße zwischen 1 fl (Femtoliter) und 1 µl (Microliter) liegt.

3. Verfahren nach Anspruch 1 oder 2,
**dadurch gekennzeichnet, dass**
die Kapillartinten nur im Randbereich des Tropfens vernetzen oder schichtbildend reagieren und dass unvernetzte oder ungebundene Bestandteile beseitigt werden.

4. Verfahren nach Anspruch 1 bis 3,
**dadurch gekennzeichnet, dass**
die Vernetzungs- oder schichtbildenden Reaktionen im Randbereich der Kapillartropfen durch Bestandteile in den Tinten oder durch den Einfluss von elektromagnetischen Wellen hervorgerufen werden.

5. Verfahren nach Anspruch 1 bis 4,
**dadurch gekennzeichnet, dass**
Reaktionen im Randbereich der Kapillartinten hervorgerufen werden durch:
- Thiol-en Reaktionen
- Cycloadditionen
- nucleophile Ringöffnungen
- Selfassembling von Molekülen und Partikeln
- selektive Faktoren an den Zellen der Kapillartinten und/oder Biotinten
- oder andere Reaktionen mit ähnlicher Wirkung sind

6. Verfahren nach Anspruch 1 bis 5,
**dadurch gekennzeichnet, dass**
die Biotinten Moleküle umfassen, die die Physiologie des gedruckten Gewebes oder Organs unterstützen.

7. Verfahren nach Anspruch 1 bis 6,
**dadurch gekennzeichnet, dass**
die Biotinten Wachstumsfaktoren, Transkriptionsfaktoren, Signalmoleküle, Markermoleküle und/oder Zielmoleküle für spätere Schlüssel-Schloss-Reaktionen enthalten.

8. Verfahren nach Anspruch 1 bis 7,
**dadurch gekennzeichnet, dass**
die gedruckten vaskulären Strukturen während des Drucks mit Nährmedium oder Blut durchströmt werden.

9. Verfahren nach Anspruch 1 bis 8,
**dadurch gekennzeichnet, dass**
die Reaktionsebene in einem Bioreaktor positioniert ist, der insbesondere während oder nach Beendigung des Drucks mit einem Blutkreislauf verbunden vorliegt

10. Verfahren nach Anspruch 1 bis 9,
**dadurch gekennzeichnet, dass**
die Vorrichtung zum Applizieren der elektromagnetischen Wellen eine UV-Lampe, eine Diode, ein Screen oder eine Laserstrahlvorrichtung ist.
